(19) Europäisches Patentamt
European Patent Office
Office européen des brevets

(11) Publication number: 0 303 348 B1

(12) # EUROPEAN PATENT SPECIFICATION

(45) Date of publication of patent specification: 08.01.92 Bulletin 92/02

(51) Int. Cl.$^5$: C07D 239/54, A61K 31/505, // C07C279/00

(21) Application number: 88306000.6

(22) Date of filing: 01.07.88

(54) 2-Amino-5-hydroxy-4-pyrimidones.

(30) Priority: 09.07.87 PCT/US87/01608

(43) Date of publication of application: 15.02.89 Bulletin 89/07

(45) Publication of the grant of the patent: 08.01.92 Bulletin 92/02

(84) Designated Contracting States: AT BE CH DE ES FR GB GR IT LI LU NL SE

(56) References cited:
EP-A- 0 063 509
DE-A- 3 009 071
DE-A- 3 436 380
GB-A- 756 189

(73) Proprietor: PFIZER INC.
235 East 42nd Street
New York, N.Y. 10017 (US)

(72) Inventor: Walker, Frederick Judson
5, Maynard Hill Road R.F.D. 1
Preston Connecticut (US)
Inventor: O'Neill, Brian Thomas
1528 Essex Road
Westbrook Connecticut (US)
Inventor: LaMattina, John Lawrence
13, Huntington Way
Ledyard Connecticut (US)

(74) Representative: Wood, David John et al
PFIZER LIMITED, Ramsgate Road
Sandwich, Kent CT13 9NJ (GB)

EP 0 303 348 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 2-amino-5-hydroxy-4-pyrimidones, pharmaceutical compositions comprising such compounds and the use of such compounds in treating inflammation or other leukotriene mediated diseases, including pulmonary, asthmatic, allergic, and gastrointestinal diseases.

European Patent Application Publication No. 0180298 refers to other 2-amino-5-hydroxy-4-pyrimidones that are stated to be useful as histamine $H_2$-antagonists.

GB-A-756189 mentions 4 : 5-dihydroxy-6-methyl-2-piperidino pyrimidine as an intermediate for making compounds useful in chemotherapy. EP-A-0063509 describes piperazino-2-pyrimidines as therapeutic agents.

The present invention relates to a compound of the formula

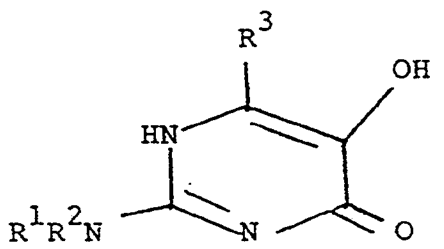

I

or a pharmaceutically acceptable salt thereof,

wherein $R^1$ is hydrogen ; $R^2$ is $(C_1\text{-}C_{15})$alkyl, $(C_3\text{-}C_{15})$alkenyl, phenyl, substituted phenyl, $(C_7\text{-}C_{20})$phenylalkyl, or substituted $(C_7\text{-}C_{20})$phenylalkyl wherein the substituents on the substituted phenyl and substituted phenylalkyl, are independently one or two of fluoro, chloro, $(C_1\text{-}C_3)$alkyl, $(C_1\text{-}C_3)$alkoxy, and trifluoromethyl ; or $R^1$ and $R^2$ together with the nitrogen atom to which they are attached form a pyrrolidinyl, substituted pyrrolidinyl, piperidyl or substituted piperidyl group, wherein the substituents on said substituted pyrrolidinyl and piperidyl groups are independently one of $(C_1\text{-}C_6)$alkyl, phenyl, and $(C_7\text{-}C_9)$phenylalkyl, and $R^3$ is H, $(C_1\text{-}C_6)$alkyl, phenyl, substituted phenyl, $(C_7\text{-}C_9)$phenylalkyl, wherein the substituents on the substituted phenyl and substituted phenylalkyl are independently one or two of fluoro, chloro, $(C_1\text{-}C_3)$alkyl, $(C_1\text{-}C_3)$alkoxy, and trifluoromethyl with the proviso that when $R^1$ and $R^2$ together with the nitrogen atom to which they are attached form a piperidyl group, $R^3$ is not methyl.

The present invention also relates to a pharmaceutical composition useful in the treatment of leukotriene mediated diseases, including inflammation, comprising an amount of a compound of the formula I effective to treat a leukotriene mediated disease and a pharmaceutically acceptable carrier.

Unless otherwise indicated, the alkyl groups of the compounds referred to herein (e.g. when $R^2$ of the compound of the formula I is $(C_1\text{-}C_{15})$ alkyl), portions of such alkyl groups and the alkyl portions of other groups (e.g., alkenyl or phenylalkyl) referred to herein may be linear, branched or cyclic. Examples of cyclic alkyl groups include cyclopentyl and cyclohexyl.

A preferred embodiment of the invention relates to compounds of the formula I wherein $R^1$ is hydrogen ; $R^2$ is linear or branched $(C_1\text{-}C_{15})$alkyl or $(C_7\text{-}C_{20})$phenylalkyl wherein the alkyl moiety is linear or branched ; and $R^3$ is hydrogen or methyl. In a more preferred embodiment, $R^1$ is hydrogen, $R^2$ is methylheptyl and $R^3$ is hydrogen. Another preferred embodiment relates to compounds of the formula I wherein $R^1$ is heptyl, $R^2$ is hydrogen and $R^3$ is methyl.

Specific preferred compounds of the present invention include the following :

2-heptylamino-5-hydroxy-6-methyl-4-pyrimidone ;
2-[(1-methylheptyl-2-amino)-5-hydroxy-4-pyrimidone ;
2-[(1-methyl-3-phenyl)-propyl]amino-5-hydroxy-4-pyrimidone ; and
2-benzylamino-5-hydroxy-4-pyrimidone.

Other compounds of the present invention include 5-hydroxy-6-methyl-2-phenylhexylamino-4-pyrimidone and 5-hydroxy-6-methyl-2-phenylpropylamino-4-pyrimidone.

Compounds of the formula I wherein $R^3$ is hydrogen may be prepared as shown below in Scheme 1, which is discussed in detail below.

Scheme 1

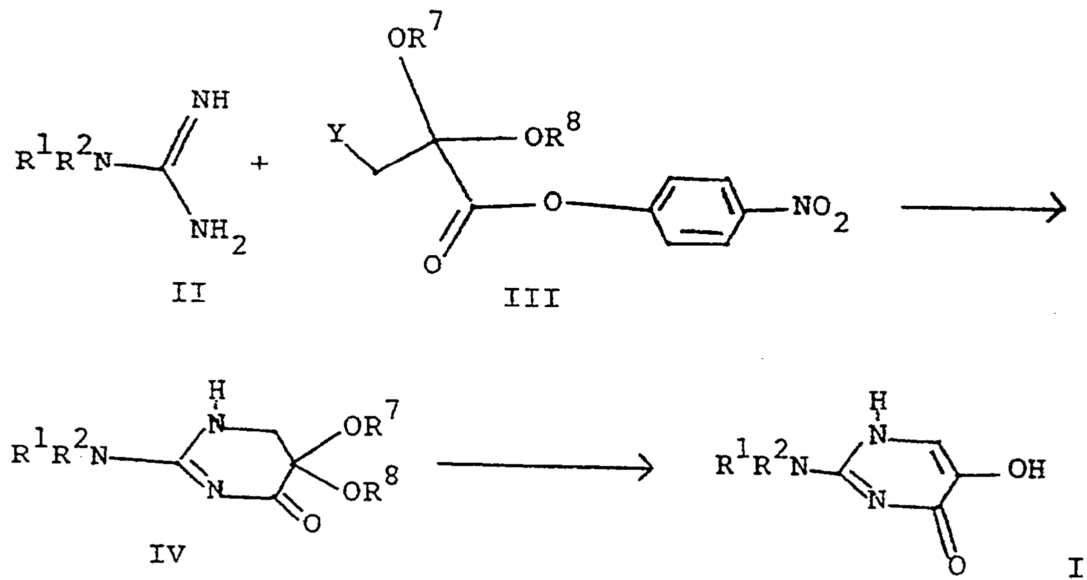

II III IV I

In order to prepare compounds of the formula I wherein $R^3$ is hydrogen, a compound of the formula II, wherein $R^1$ and $R^2$ are as defined above, may be reacted with a compound of the formula III (prepared as described by J. LaMattina, Tetrahedron Letters, 2053 (1983)), wherein Y is chloro, bromo or iodo, preferably bromo, and $R^7$ and $R^8$ are independently selected from $C_1$-$C_4$ alkyl (e.g. ethyl), preferably in a polar protic solvent and under an inert atmosphere, in the presence of an inorganic base (e.g., sodium hydroxide, potassium hydroxide or sodium hydride) or a strong organic base (e.g., sodium ethoxide or potassium tert-butoxide), at a temperature of about 0 to about 100°C. The preferred base is sodium ethoxide. The reaction mixture is preferably maintained at a temperature of about 25°C for about 30 minutes to about 24 hours, more preferably for about 1 hour. Preferred solvents are $C_1$ to $C_4$ alcohols. The resulting compound of the formula IV is reacted with an acid catalyst (e.g., hydrochloric acid) in a polar protic solvent at a temperature of about 0 to 100°C (preferably, about 60°C) for about 4 to about 48 hours to privide a compound of the formula I. Preferably, the acid catalysed reaction is carried out in neat formic acid.

Compounds of the formula I wherein $R^3$ is other than hydrogen may be prepared as shown in Scheme 2, which is discussed in detail below.

Scheme 2

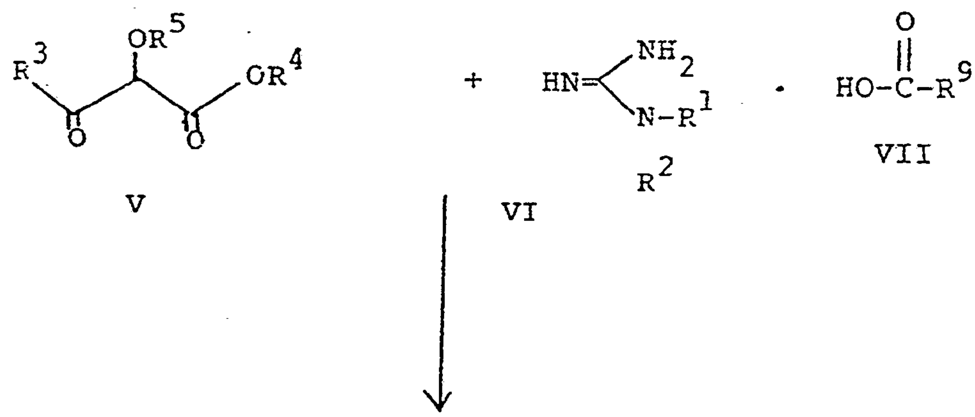

V + VI · VII

VIII* + IX*

* formula shown on next page

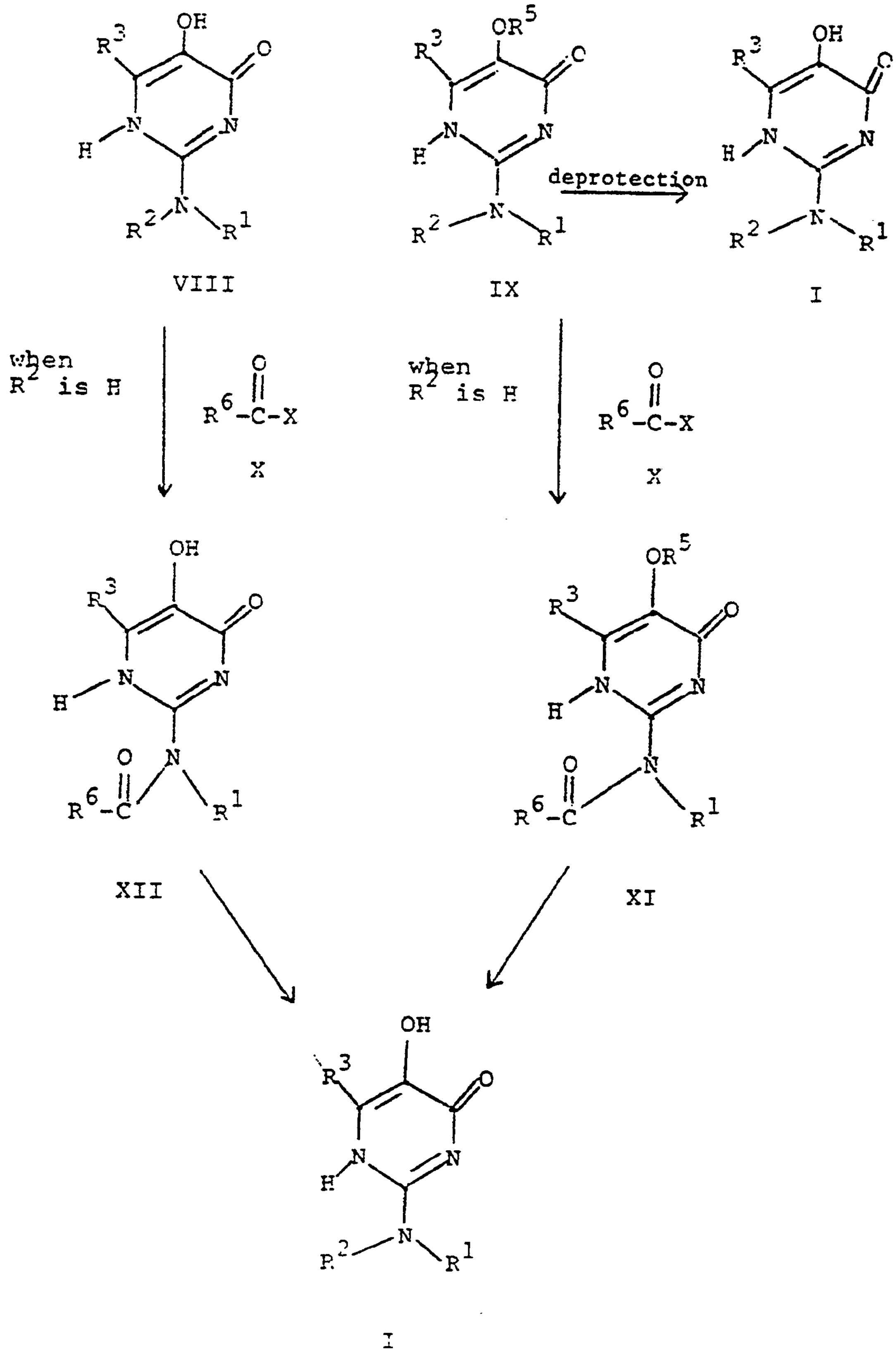


VIII IX I

XII XI

I

A compound of the formula V wherein $R^3$ is as defined for formula I, $R^4$ is $C_1$-$C_4$ alkyl,

and $R^5$ is a protecting group (e.g., $-\overset{O}{\overset{\|}{C}}-CH_3$, 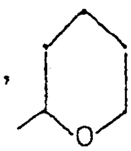,

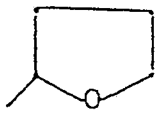 or $-CH_2-$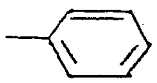 )

is reacted with a guanidine salt of the compound of the formula VI (e.g., a salt formed by the compound of the formula VI with a compound of the formula VII wherein $R^9$ is $C_1$-$C_4$ alkyl), preferably an acetate,salt. The foregoing reaction provides a pyrimidone of the formula IX. When $R^5$ is an acetate group, the foregoing reaction provides a mixture of pyrimidones of the formulae VIII and IX. The foregoing reaction may be carried out in an aprotic solvent (e.g., dimethylformamide) at a temperature of about 25 to about 150°C, preferably about 100°C, for about 1 to about 48 hours, preferably about 24 hours.

Although both the compound of the formula VIII and the compound of the formula IX may be reacted with an activated ester to prepare a compound of the formula I (via an intermediate of the formula XII or XI, respectively) as described below, it is, generally, convenient to convert the compound of the formula VIII to the compound of the formula IX, before reacting the latter with the activated ester.

In order to convert the compound of the formula VIII to a compound of the formula IX, the compound of the formula VIII is treated with a reagent capable of introducing the $R^5$ protecting group. For example, the compound of the formula VIII may be reacted with acetic anhydride and a base (e.g., pyridine or triethylamine), with or without an added solvent (e.g., methylene chloride), for example at a temperature of about −20 to about−50°C, preferably about 25°C, to obtain the compound of the formula IX.

The compound of the formula IX or the compound of the formula VIII, or the mixture of the formula IX and the compound of the formula VIII, is reacted with an activated ester of the formula X, wherein $R^6$ is hydrogen, ($C_1$-$C_{14}$)alkyl, $C_2$-$C_{14}$(alkenyl), ($C_6$-$C_{19}$)-phenylalkyl, or substituted ($C_6$-$C_{19}$)phenylalkyl, and wherein the substituents on the substituted phenylalkyl are independently one or two of fluoro, chloro, ($C_1$-$C_3$)alkyl, ($C_1$-$C_3$)alkoxy and trifluoromethyl, and the group X is chloro or bromo, preferably chloro, for example in an aprotic solvent (preferably methylene chloride or pyridine) at about −20 to about 100°C (preferably about 0°C) for about 0.5 to about 24 hours (preferably about 18 hours) with a base catalyst (preferably, dimethylaminopyridine). As a result, there is formed, respectively, the compound of the formula XI, the compound of the formula XII, or a mixture of the compound of the formula XI and the compound of the formula XII.

The compound of the formula XI or the compound of the formula XII may be reduced with a hydride reducing agent (e.g., diborane) in an anhydrous solvent (preferably tetrahydrofuran), generally for about 1 to about 24 hours (preferably about 18 hours) generally at about 0 to about 50°C (preferably about 25°C) to prepare the compound of the formula I. If reduction of the group

$$R^6-\overset{O}{\overset{\|}{C}}-$$

to form an $R^2$ group does not also deprotect the $R^5$ group in the compound of the formula XI (such as occurs when $R^5$ is acyl), a further deprotecting step (e.g. hydrogenation when $R^5$ is benzyl or treatment with aqueous acid when $R^5$ is tetrahydropyranyl) provides the compound of the formula I.

The compound of the formula IX may also be deprotected to provide the compound of the formula I. When

$$R^5 \text{ is } -\overset{O}{\overset{\|}{C}}-CH_3,$$

deprotection may be effected by hydrolysis ; when $R^5$ is tetrahydropyranyl, deprotection may be effected by treatment with aqueous acid ; and when $R^5$ is

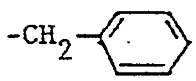 ,

deprotection may be effected by hydrogenation.

Acid addition salts of the compounds of formula I are prepared in a conventional manner by treating a solution or suspension of the free base of a compound of the formula I with about one chemical equivalent of a pharmaceutically acceptable acid. Conventional concentration and recrystallisation techniques are employed in isolating the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroidic, sulfamic, sulfonic such as methanesulfonic, benzenesulfonic, and related acids. Preferably, the acid is phosphoric acid.

Base addition salts of compounds of the formula I may be prepared in a conventional manner by reacting a compound of the formula I with about one chemical equivalent of inorganic base such as an alkali metal hydroxide or an alkaline earth metal hydroxide.

The activity of the compounds of formula I and their salts in the treatment of pulmonary, asthmatic, allergic and inflammatory diseases may be determined by a standard test measuring an agent's ability to inhibit cyclooxygenase and 5-lipoxygenase enzyme activity of rat basophil leukemia 5-(RBL-1) cells. According to this test as described by Jakschick et al., Prostaglandins, 16,733-747 (1978), a monolayer of RBL-1 cells is grown for 1 or 2 days in spinner culture in Eagle's minimum essential medium, 15% heat-inactivated fetal calf serum and an antibiotic/antimycotic mixture. The cells are washed after centrifugation and incubated in a buffer. A volume of 0.5 ml of cell suspension is preincubated at 30°C for ten minutes with a 1 microliter dimethylsulfoxide (DMSO) solution of the agent to be tested. The incubation is initiated by simultaneous addition of 5 microliters of ($^{14}C$)-arachidonic acid in ethanol and 2 microliters calcium ionophore (A-21387) in DMSO for final concentrations of 5 and 7.6 micromolar, respectively. Five minutes later, the incubation is terminated by the addition of 0.27 ml acetonitrile/acetic acid (100 :3). High pressure liquid chromatography is performed using acetonitrile/water/acetic acid solvent. Radiolabeled prostaglandin $D_2$($PGD_2$), leukotriene $B_4$($LTB_4$), 5-hydroxyeicosatetraenoic acid (5-HETE), and unreacted arachidonic acid are determined. The inhibitory effect on the cyclooxygenase pathway is assessed from the reduction of $PGD_2$ levels and the inhibitory effect on the 5-lipoxygenase pathway is assessed from the decrease in the amount of $LTB_4$ and 5-HETE.

The compounds of the formula I and their pharmaceutically acceptable salts are effective inhibitors of mammalian leukotriene biosynthesis in mammals and are thus useful in the treatment of various leukotriene mediated conditions. In particular, the compounds have utility, both as the sole active agent and also in combination with other active agents, for the treatment of various pulmonary, gastrointestinal, inflammatory, dermatological and cardiovascular conditions such as inflammation, arthritis, allergy, psoriasis, asthma, bronchitis, pulmonary hypertension and hypoxia, peptic ulcers, inflammatory bowel disease or cardiovascular spasm, such as acute myocardial infarctions, and the like in mammals, especially in humans.

For treatment of the various conditions described above, the compounds of formula I and their pharmaceutically acceptable salts may be administered to a subject in need of treatment by a variety of conventional routes of administration, including oral, by injection, topical, and in an aerosol carrier composition for administration by inhalation.

The exact dosage of a compound of the present invention will depend upon such factors as the age, weight and condition of the patient and the severity of disease. In general, however, a therapeutically-effective dose of a compound of formula I or a pharmaceutically acceptable salt thereof will range from 0.01 to 100 mg/kg body weight of the subject to be treated per day, preferably 0.1 to 50 mg/kg per day.

Although the compounds of formula I and their salts can be administered alone, they will generally be administered in admixture with a pharmaceutical carrier selected with regard to the intended route of administration and standard pharmaceutical practice. For example, oral administration may be in the form of tablets containing such excipients as starch or lactose, or in the form of elixirs or suspensions containing flavoring or coloring agents. In the case of animals, they are advantageously contained in an animal feed or drinking water. For parenteral injection, they may be used in the form of a sterile aqueous solution which may contain other solutes, for example enough salt or glucose to make the solution isotonic. Other active compounds, including NSAIDS (non-steroidal antiinflammatory drugs) may be administered along with the compounds of the present invention.

The following non-limiting Examples are illustrative of the compounds of the present invention. All melting points referred to in the Examples are uncorrected.

Example 1

2-Heptylamino-5-hydroxy-6-methyl-4-pyrimidone

A. 5-Acetoxy-2-amino-6-methyl-4-pyrimidone

2-Amino-5-hydroxy-6-methyl-4-pyrimidone (0.88 g, prepared as described in Hull, J. Chem. Soc., 2033 (1956)), was treated with pyridine (5 ml) and acetic anhydride (0.5 ml) at 0°C. The reaction mixture was stirred at 25°C for 18 hours. Filtration provided a white solid (0.64 g). Recrystallization (absolute ethanol) afforded a white crystalline solid (m.p. : 273-276°C).

B. 2-Heptylamino-5-hydroxy-6-methyl-4-pyrimidone

A sample prepared as above (1.83 g, 10 mmol) in pyridine (25 ml) was cooled 0°C under $N_2$ and treated with 4-dimethylaminopyridine (1.6 g) followed by the dropwise addition of heptanoyl chloride (1.9 g, 10 mmol) in methylene chloride (10 ml). Aqueous extractive work up gave a crude product which was used directly. This product (0.5 g, 1.7 mmol) was dissolved in dry tetrahydrofuran (THF) (10 ml) and treated with borane-THF (1M, 8 ml). The reaction mixture was stirred overnight and was then quenched with methanol (10 ml) and concentrated on a rotary evaporator. The residue was refluxed in 20 ml of 6N HCl for 2 hours. The cooled reaction mixture was filtered, providing a white crystalline product (m.p. : 233-235°C).

Example 2

2-(6-Methylheptyl-2-amino)-5-hydroxy-4-pyrimidone

A. 2-N-Guanidino-6-methylheptane Acetate

3,5-Dimethylpyrazole-1-carboxamidine (10 g, 0.07 mol), 2-amino-6-methylheptane (9.33 g, 0.07 mol) and acetic acid (4.5 ml) were combined and heated under reflux for 96 hours. The reaction mixture was cooled and filtered, yielding a white solid (12.6 g, 82%) m.p. 148-152°C.

B. 2-(6-Methylheptyl-2-amino)-5,5-diethoxy-6H-4-pyrimidone

The above N-alkyl guanidine (5.75 g, 25 mmol) was treated with freshly prepared sodium ethoxide (25 mmol) at 25°C for 1 hour under $N_2$. The reaction was filtered and the filtrate was added to a solution (50 ml ethanol) of 4-nitrophenyl 3-bromo-2,2-diethoxypropionate (3.0 g, 8.3 mmol). The reaction mixture was heated under reflux for 24 hours and was then cooled and concentrated. The residue was dissolved in ethyl acetate (100 ml) and extracted with 20% sodium carbonate (2 × 50 ml). The organic layer was dried (sodium sulfate) and concentrated, yielding 1.91 g (74%) of crude product. Chromatography (silica gel, 10 : 1, chloroform : methanol as eluent) and recrystallization (acetonitrile) afforded an analytically pure sample, m.p. 170-172°C. Anal. Calcd. for $C_{16}H_{33}N_3O_4$ : C, 60.81 ; H, 9.99 ; N, 13.41. Found : C, 60.44 ; H, 9.98 ; N, 13.21.

C. 2-(6-Methylheptyl-2-amino)-5-hydroxy-4-pyrimidone

The above compound (1.0 g, 3.2 mmol) was heated under reflux in formic acid (50 ml) for 24 hours. The reaction mixture was then cooled and concentrated. Chromatography on silica gel (chloroform :methanol, 20: 1) afforded a solid (0.26 g, 34%) m.p. 95-100°C. Trituration of the solid with ethanol and recrystallization (acetonitrile) afforded an analytically pure sample. Anal. Calcd. for $C_{12}H_{21}N_3O_2$ : C, 60.22 ; H, 8.84 ; N, 17.56. Found : C, 59.78 ; H, 8.95 ; N, 17.31.

Example 3

The following compounds were prepared using, as indicated below, a method similar to that set forth in Example 1 or 2 :

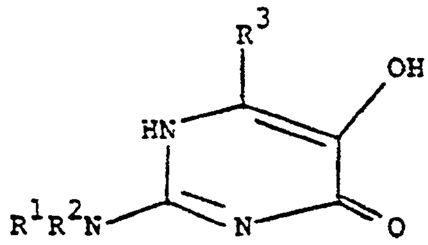

| $R^1$ | $R^2$ | $R^3$ | m.p. | Example |
|---|---|---|---|---|
| $(CH_2)_5CH_3$ | H | H | 237-240°C | 1 |
| $(CH_2)_6CH_3$ | H | $CH_3$ | 233-235°C | 1 |
| $CH_3(CH)CH_2CH_2Ph$ | H | H | 125-126°C | 2 |
| $CH_2Ph$ | H | H | 210-215°C | 1 |
| $CH_3(CH)(CH_2)_3CH(CH_3)_2$ | H | H | 95-100°C | 2 |

Ph = phenyl

Example 4

Using the method of Jakschick et al. described above, the title compounds of Example 1 and 2 and the compounds of Example 3 were tested for their ability to inhibit lipoxygenase enzyme.

All of the compounds were active at a level of 20 micromolar.

## Claims

**Claims for the following Contracting States : AT, BE, CH, DE, FR, GB, IT, LI, LU, NL, SE**

1. A compound of the formula

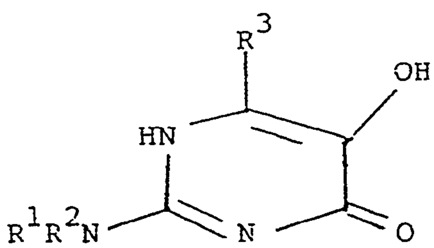

or a pharmaceutically acceptable salt thereof,

wherein $R^1$ is hydrogen ; $R^2$ is $(C_1-C_{15})$alkyl, $(C_3-C_{15})$alkenyl, phenyl, substituted phenyl, $(C_7-C_{20})$phenylalkyl, or substituted $(C_7-C_{20})$phenylalkyl, wherein the substituents on the substituted phenyl and substituted phenylalkyl are independently one or two of fluoro, chloro, $(C_1-C_3)$alkyl, $(C_1-C_3)$alkoxy, and trifluoromethyl ; or $R^1$ and $R^2$ together with the nitrogen atom to which they are attached form a pyrrolidinyl, substituted pyrrolidinyl, piperidyl or substituted group, wherein the substituents on said substituted pyrrolidinyl and piperidyl groups are independently one of $(C_1-C_6)$alkyl, phenyl, and $(C_7-C_9)$phenylalkyl ; and $R^3$ is hydrogen, $(C_1-C_6)$alkyl, phenyl, substituted phenyl, $(C_7-C_9)$phenyl-alkyl, or substituted $(C_7-C_9)$phenylalkyl, wherein the substituents on the substituted phenyl and substituted phenylalkyl are independently one or two of fluoro, chloro, $(C_1-C_3)$alkyl, $(C_1-C_3)$alkoxy, and trifluoromethyl, wherein said alkyl groups, portions of such alkyl groups and the alkyl portions of other groups may be linear, branched or cyclic, with the proviso that when $R^1$ and $R^2$ together with the nitrogen atom to which they are attached form a piperidyl group, $R^3$ is not methyl.

2. A compound according to claim 1, wherein $R^1$ is hydrogen, $R^2$ is linear or branched $(C_1-C_{15})$alkyl or $(C_7-C_{20})$phenylalkyl wherein the alkyl moiety is linear or branched ; and $R^3$ is hydrogen or methyl.

3. A compound according to claim 1, wherein $R^2$ is cyclopentyl or cyclohexyl.

4. A compound according to claim 1 which is
2-heptylamino-5-hydroxy-6-methyl-4-pyrimidone ;
2-(6-methylheptyl-2-amino)-5-hydroxy-4-pyrimidone ;
[2-[(1-methyl-3-phenyl)-propyl]amino-5-hydroxy-4-pyrimidone ; or
2-benzylamino-5-hydroxy-4-pyrimidone.

5. A pharmaceutical composition which comprises a compound according to any one of the preceding claims or a pharmaceutically acceptable salt thereof, and a pharmaceutically acceptable diluent or carrier.

6. A compound as claimed in any one of claims 1 to 4, or a pharmaceutically acceptable salt thereof, for use as a medicament.

7. The use of a compound as defined in any one of claims 1 to 4, without the proviso, or of a pharmaceutically acceptable salt thereof, for the manufacture of a medicament for the treatment of leukotriene-mediated pulmonary, asthmatic, allergic, cardiovascular, gastrointestinal or inflammatory diseases.

8. The use of claim 7, wherein said diseases are arthritis, psoriasis, bronchitis, pulmonary hypertension, pulmonary hypoxia, peptic ulcers, inflammatory bowel disease or cardiovascular spasm.

**Claims dor the following Contracting States : ES, GR**

1. A process for preparating a compound of the formula

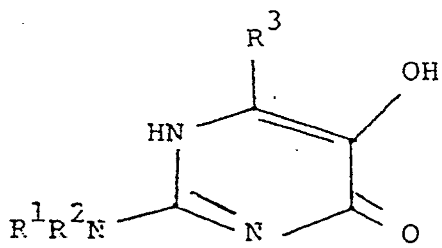

I

or a pharmaceutically acceptable salt thereof,

wherein $R^1$ is hydrogen ; $R^2$ is $(C_1\text{-}C_{15})$alkyl, $(C_3\text{-}C_{15})$alkenyl, phenyl, substituted phenyl, $(C_7\text{-}C_{20})$phenylalkyl, or substituted $(C_7\text{-}C_{20})$phenylalkyl, wherein the substituents on the substituted phenyl and substituted phenylalkyl are independently one or two of fluoro, chloro, $(C_1\text{-}C_3)$alkyl, $(C_1\text{-}C_3)$alkoxy, and trifluoromethyl ; or $R^1$ and $R^2$ together with the nitrogen atom to which they are attached form a pyrrolidinyl, substituted pyrrolidinyl, piperidyl or substituted piperidyl group, wherein the substituents on said substituted pyrrolidinyl and piperidyl groups are independently one of $(C_1\text{-}C_6)$alkyl, phenyl, and $(C_7\text{-}C_9)$phenylalkyl ; and $R^3$ is hydrogen ; wherein said alkyl groups, portions of such alkyl groups and the alkyl portions of other groups may be linear, branched or cyclic, comporising reacting a compound of the formula

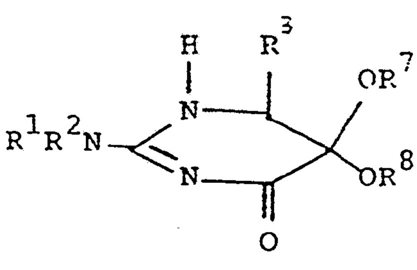

IV

wherein $R^3$ is hydrogen, $R^1$ and $R^2$ are as defined above, and $R^7$ and $R^8$ are independently selected from $(C_1\text{-}C_4)$alkyl with an acid catalyst, and, if desired, preparing the acid addition salt.

2. A process according to claim 1, wherein said compound of formula IV is obtained by reacting a compound of the formula

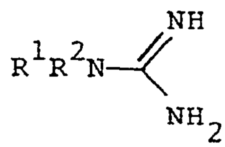

wherein $R^1$ and $R^2$ are as defined above with a compound of the formula

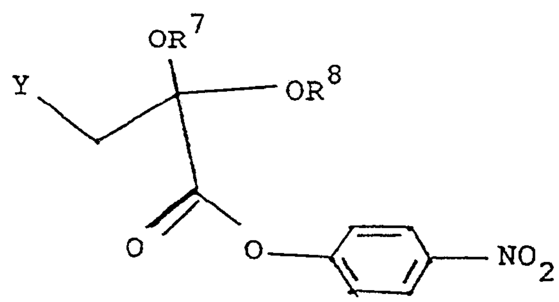

III

wherein $R^7$ and $R^8$ are as defined above and Y is chloro, bromo or iodo.

3. A process for preparing a compound of the formula

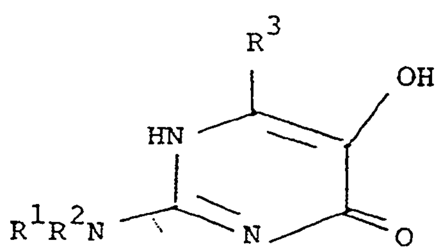

I

or a pharmaceutically acceptable salt thereof,

wherein $R^1$ and $R^2$ are as defined in claim 1 ; and $R^3$ is hydrogen, $(C_1\text{-}C_6)$alkyl, phenyl, substituted phenyl, $(C_7\text{-}C_9)$phenyl-alkyl, or substituted $(C_7\text{-}C_9)$phenylalkyl, wherein the substituents on the substituted phenyl and substituted phenylalkyl are independently one or two of fluoro, chloro, $(C_1\text{-}C_3)$alkyl, $(C_1\text{-}C_3)$alkoxy, and trifluoromethyl comprising reducing, with a hydride reducing agent in an anhydrous solvent, a compound of the formula

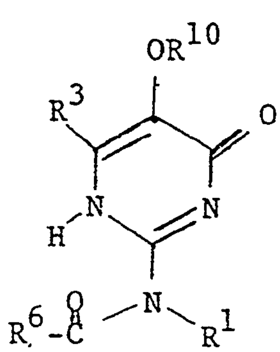

XIII

wherein $R^1$ is hydrogen, $R^3$ is as defined above, $R^{10}$ is hydrogen or $R^5$, $R^5$ is a protecting group and $R^6$ is hydrogen, $(C_1\text{-}C_{14})$alkyl, $(C_2\text{-}C_{14})$alkenyl, $(C_6\text{-}C_{19})$phenylalkyl or substituted $(C_6\text{-}C_{19})$-phenylalkyl, wherein the substituents on the substituted phenylalkyl are independently one or two of fluoro, chloro, $(C_1\text{-}C_3)$alkyl, $(C_1\text{-}C_3)$alkoxy and trifluoromethyl, and if $OR^5$ is a group that is not converted by reduction to a hydroxyl group, deprotecting the $R^5$ group after the reduction reaction so that $OR^5$ is converted to hydroxyl, and if desired preparing the acid addition salt ; wherein said alkyl groups, portions of such alkyl groups and the alkyl portions of other groups may be linear, branched or cyclic.

4. A process according to claim 3, wherein said compound of the formula XIII is prepared by reacting a

compound of the formula

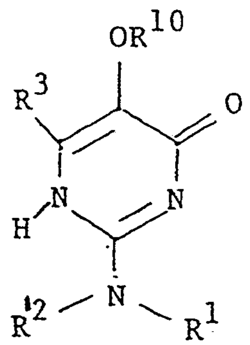

XIV

wherein $R^1$, $R^3$ and $R^{10}$ are as defined in claim 3 and $R^{12}$ is H with a compound of the formula

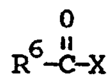

X

wherein $R^6$ is as defined in claim 3 and X is chloro or bromo.

5. A process according to claim 4, wherein said compound of the formula XIV wherein $R^{10}$ is $R^5$ is prepared by reacting a compound of the formula

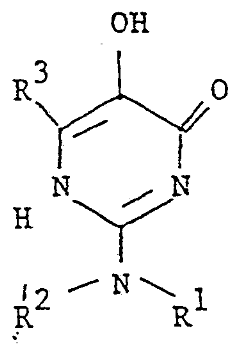

VIII

wherein $R^1$ and $R^3$ are as defined in claim 3 and $R^{12}$ is H with a reagent capable of replacing the hydrogen in the hydroxyl group with a protecting group.

6. A process according to claim 5, wherein said compound of the formula XIV wherein $R^{10}$ is $R^5$ is prepared by reacting said compound of the formula VIII with acetic anhydride and a base.

7. A process according to any one of claims 4-6, wherein said compound of the formula XIV is obtained by reacting a compound of the formula

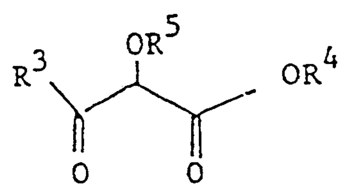

V

wherein $R^3$ and $R^5$ are as defined in claim 3 and $R^4$ is $C_1$-$C_4$ alkyl, with a salt of a compound of the formula

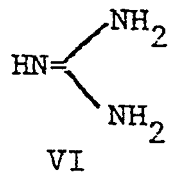

VI

8. A process according to claim 7, wherein said salt is a compound of the formula

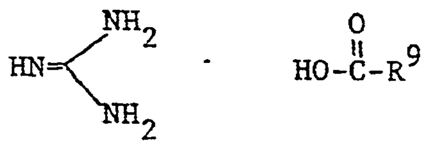

wherein $R^9$ is $(C_1\text{-}C_4)$alkyl.

9. A process for preparing a compound of the formula

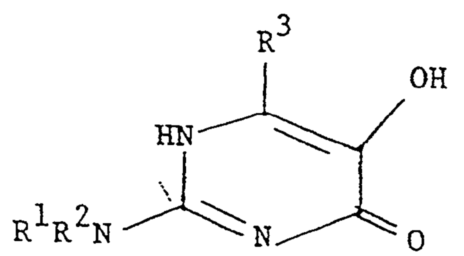

I

or a pharmaceutically acceptable salt thereof,

wherein $R^1$, $R^2$ and $R^3$ are as defined in claim 3 comprising deprotecting a compound of the formula

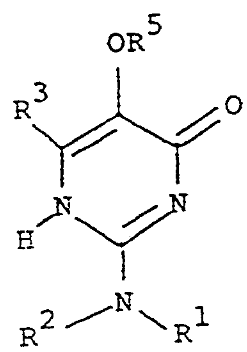

IX

wherein $R^1$, $R^2$ and $R^3$ are as defined above, and $R^5$ is a protecting group, and if desired preparing the acid addition salt.

10. A process according to claim 9, wherein the compound of the formula IX is deprotected by (a) hydrolysis; (b) treatment with aqueous acid ; or (c) hydrogenation.

11. A process according to any one of the preceding claims wherein $R^1$ is hydrogen, and $R^2$ is linear or branched $(C_1\text{-}C_{15})$alkyl or $(C_7\text{-}C_{20})$phenylalkyl wherein the alkyl moiety is linear or branched.

12. A process according to any one of claims 1 to 10, wherein $R^2$ is cyclopentyl or cyclohexyl.

13. A process according to any one of the preceding claims wherein said compound of the formula I is selected from the group consisting of

2-heptylamino-5-hydroxy-6-methyl-4-pyrimidone ;

2-(6-methylheptyl-2-amino)-5-hydroxy-4-pyrimidone ;

2-[1-methyl-3-phenyl)-propyl]amino-5-hydroxy-4-pyrimidone ; and

2-benzylamino-5-hydroxy-4-pyrimidone.

## Patentansprüche

### Patentansprüche für die folgenden Vertragsstaaten : AT, BE, CH, DE, FR, GB, IT, LI, LU, NL, SE

1. Verbindung der Formel

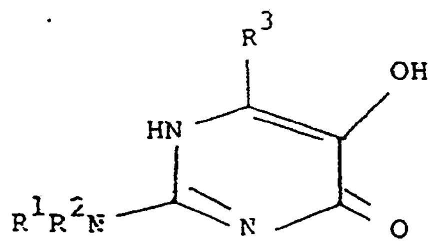

oder eines pharmazeutisch annehmbaren Salzes davon, worin $R^1$ Wasserstoff ist, $R^2$ $(C_1-C_{15})$-Alkyl, $(C_3-C_{15})$-Alkenyl, Phenyl, substistuiertes Phenyl, $(C_7-C_{20})$-Phenylalkyl oder substituiertes $(C_7-C_{20})$-Phenylalkyl ist, wobei die Substituenten des substituierten Phenyl und substituerten Phenylalkyls unabhängig voneinander ein oder zwei Reste aus der Gruppe Fluor, Chlor, $(C_1-C_3)$-Alkyl, $(C_1-C_3)$-Alkoxy und Trifluormethyl sind, oder $R^1$ und $R^2$ zusammen mit dem Stickstoffatom, an das sie gebunden sind, eine Pyrrolidinyl-, substituierte Pyrrolidinyl-, Piperidyl- oder substituierte Piperidylgruppe bedeuten, wobei die Substituenten der substituierten Pyrrolidinyl- und Piperidylgruppen unabhängig voneinander ein Reit aus der Gruppe $(C_1-C_6)$Alkyl ; Phenyl und $(C_7-C_9)$-Phenylalkyl sind, und $R^3$ Wasserstoff, $(C_1-C_6)$-Alkyl, Phenyl, substituiertes Phenyl, $(C_7-C_9)$-Phenylalkyl oder substituiertes $(C_7-C_9)$-Phenylalkyl ist, wobei die Substituenten des substituierten Phenyls und substituierten Phenylalkyls unabhängig voneinander ein oder zwei Reste aus der Gruppe Fluor, Chlor, $(C_1-C_3)$-Alkyl, $(C_1-C_3)$-Alkoxy und Trifluormethyl sind, wobei die Alkylgruppen, Teile der Alkylgruppen und die Alkylteile anderer Gruppen linear, verzweigt oder cyclisch sein können, mit der Maßgabe, daß, wenn $R^1$ und $R^2$ zusammen mit dem Stickstoffatom, an das sie gebunden sind, eine Piperidylgruppe bilden, $R^3$ nicht Methyl ist.

2. Verbindung nach Anspruch 1, worin $R^1$ Wasserstoff ist, $R^2$ lineares oder verzweigtes $(C_1-C_{15})$-Alkyl oder $(C_7-C_{20})$-Phenyl-alkyl, wobei der Alkylteil linear oder verzweigt ist, ist und $R^3$ Wasserstoff oder Methyl ist.

3. Verbindung nach Anspruch 1, worin $R^2$ Cyclopentyl oder Cyclohexyl ist.

4. Verbindung nach Anspruch 1, welche
2-Heptylamino-5-hydroxy-6-methyl-4-pyrimidon ;
2-(6-Methylheptyl-2-amino)-5-hydroxy-4-pyrimidon ;
2-[1-Methyl-3-phenyl)-propyl]amino-5-hydroxy-4-pyrimidon ;
oder 2-Benzylamino-5-hydroxy-4-pyrimidon ist.

5. Pharmazeutisches Präparat, welches aus einer Verbindung nach einem der vorhergehenden Ansprüche oder einem pharmazeutisch annehmbaren Salz davon und einem pharmazeutisch annehmbaren Verdünnungsmittel oder Träger zusammengesetzt ist.

6. Verbindung nach einem der Ansprüche 1 bis 4, oder ein pharmazeutisch annehmbares Salz davon, zur Verwendung als Medikament.

7. Verwendung einer Verbindung nach einem der Ansprüche 1 bis 4, ohne der Maßgabe, oder eines pharmazeutisch annehmbaren Salzes davon, zur Herstellung eines Medikamentes zur Behandlung von leukotrienvermittelter Lungen-, asthmatischer, allergischer, kordiovaskulärer, gastrointestinaler oder Entzündungs-Erkrankungen.

8. Verwendung nach Anspruch 7, wobei die Krankheiten Arthritis, Psoriasis, Bronchitis, Lungenhypertension, Lungenhypoxie, Magengeschwüre, Darmentzündungen oder kardiovaskulärer Spasmus sind.

### Patentansprüche für die folgenden Vertragsstaaten : ES, GR

1. Verfahren zur Herstellung einer Verbindung der Formel

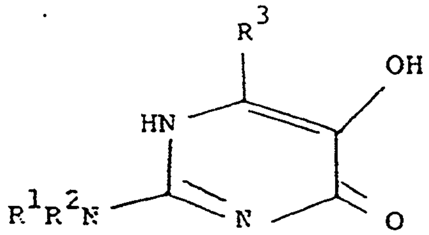

I

oder eines pharmazeutisch annehmbaren Salzes davon, worin $R^1$ Wasserstoff ist, $R^2$ ($C_1$-$C_{15}$)-Alkyl, ($C_3$-$C_{15}$)-Alkenyl, Phenyl, substistuiertes Phenyl, ($C_7$-$C_{20}$)-Phenylalkyl oder substituiertes ($C_7$-$C_{20}$)-Phenylalkyl ist, wobei die Substituenten des substituierten Phenyl und substituerten Phenylalkyls unabhängig voneinander ein oder zwei Reste aus der Gruppe Fluor, Chlor, ($C_1$-$C_3$)-Alkyl, ($C_1$-$C_3$)-Alkoxy und Trifluormethyl sind, oder $R^1$ und $R^2$ zusammen mit dem Stickstoffatom, an das sie gebunden.sind, eine Pyrrolidinyl-, substituierte Pyrrolidinyl-, Piperidyl- oder substituierte Piperidylgruppe bedeuten, wobei die Substituenten der substituierten Pyrrolidinyl- und Piperidylgruppen unabhängig voneinander ein Rest aus der Gruppe ($C_1$-$C_6$)-Alkyl, Phenyl und ($C_7$-$C_9$)-Phenylalkyl sind, und $R^3$ Wasserstoff ist ; wobei die Alkylgruppen, Teile der Alkylgruppen und die Alkylteile anderer Gruppen linear, verzweigt oder cyclisch sein können, wobei eine Verbindung der Formel

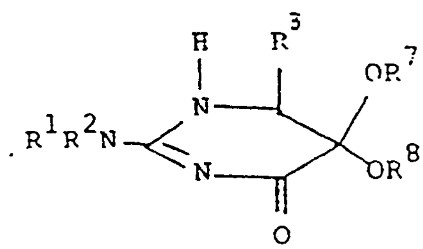

IV,

worin $R^3$ Wasserstoff ist, $R^1$ und $R^2$ obige Bedeutung haben und $R^7$ und $R^8$ unabhängig voneinander aus ($C_1$-$C_4$)-Alkyl ausgewählt sind, mit einem Säurekatalysator zur Reaktion gebracht, und, falls gewünscht, das Säureadditionssalz hergestellt wird.

2. Verfahren nach Anspruch 1, worin die Verbindung der Formel IV durch Umsetzung einer Verbindung der Formel

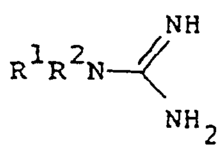

,

worin $R^1$ und $R^2$ obige Bedeutung haben mit einer Verbindung der Formel

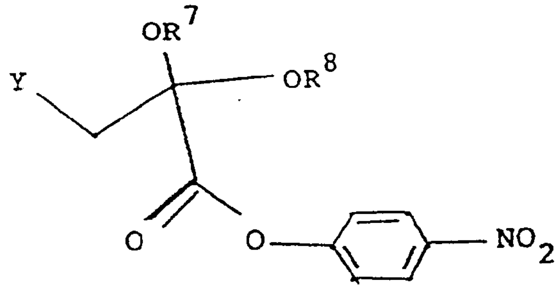

III,

worin $R^7$ und $R^8$ obige Bedeutung haben und Y Chlor, Brom oder Jod ist, erhalten wird.

3. Verfahren zur Herstellung einer Verbindung der Formel

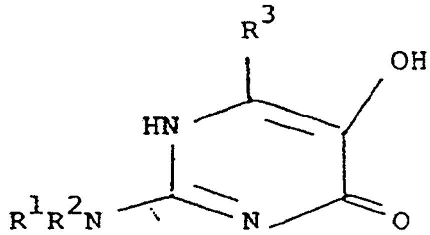

I

oder eines pharmazeutischen Salzes davon, worin $R^1$ und $R^2$ die in Anspruch 1 angegebene Bedeutung haben; und $R^3$ Wasserstoff, $(C_1\text{-}C_6)$-Alkyl, Phenyl, substituiertes Phenyl, $(C_7\text{-}C_9)$-Phenylalkyl oder substituiertes$(C_7\text{-}C_9)$-Phenylalkyl ist, wobei die Substituenten des substituierten Phenyls und substituierten Phenylalkyls unabhängig voneinander ein oder zwei Reste aus der Gruppe Fluor, Chlor, $(C_1\text{-}C_3)$-Alkyl, $(C_1\text{-}C_3)$-Alkoxy und Trifluormethyl sind, wobei eine Verbindung der Formel

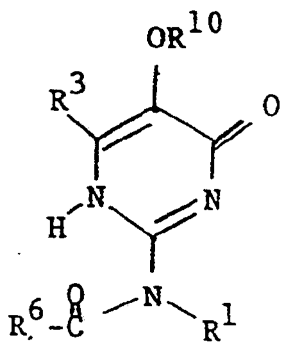

XIII,

worin $R^1$ Wasserstoff ist, $R^3$ obige Bedeutung hat, $R^{10}$ Wasserstoff oder $R^5$ ist, $R^5$ eine Schutzgruppe ist und $R^6$ Wasserstoff, $(C_1\text{-}C_{14})$-Alkyl, $(C_2\text{-}C_{14})$-Alkenyl, $(C_6\text{-}C_{19})$-Phenyl-alkyl oder substituiertes $(C_6\text{-}C_{19})$-Phenylalkyl ist, wobei die Substituenten des substituierten Phenylalkyls unabhängig voneinander ein oder zwei Reste aus der Gruppe Fluor, Chlor, $(C_1\text{-}C_3)$-Alkyl, $(C_1\text{-}C_3)$-Alkoxy und Trifluormethyl sind, mit einem Hydrid-Reduktionsmittel reduziert wird, und falls $OR^5$ eine Gruppe ist, welche durch die Reduktion nicht in eine Hydroxylgruppe übergeführt wird, nach der Reduktion die $R^5$-Gruppe entfernt wird, sodaß $OR^5$ in eine Hydroxylgruppe übergeführt wird ; wobei die Alkylgruppen, Teile solcher Alkylgruppen und die Alkylteile anderer Gruppen linear, verzweigt oder cyclisch sein können.

4. Verfahren nach Anspruch 3, wobei die Verbindung der Formel XIII durch Reaktion einer Verbindung der Formel

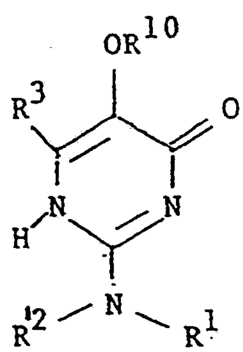

XIV,

worin $R^1$, $R^3$ und $R^{10}$ die in Anspruch 3 angegebene Bedeutung haben und $R^{12}$ H ist, mit einer Verbindung der Formel

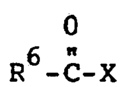

X,

worin $R^6$ die in Anspruch 3 angegebene Bedeutung hat und X Chlor oder Brom ist, hergestellt wird.

5. Verfahren nach Anspruch 4, wobei die Verbindung der Formel XIV, worin $R^{10}$ $R^5$ ist, durch Reaktion einer Verbindung der Formel

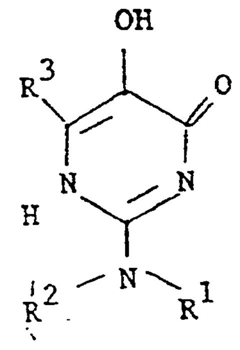

VIII,

worin $R^1$ und $R^3$ die in Anspruch 3 angegebene Bedeutung haben und $R^{12}$ H ist, mit einem Reagens, das geeignet ist den Wasserstoff in der Hydroxylgruppe durch eine Schutzgruppe zu ersetzen, hergestellt wird.

6. Verfahren nach Anspruch 5, wobei die Verbindung der Formel XIV, worin $R^{10}$ $R^5$ ist, durch Reaktion der Verbindung der Formel VIII mit Essigsäureanhydrid und einer Base hergestellt wird.

7. Verfahren nach einem der Ansprüche 4 bis 6, wobei die Verbindung der Formel XIV durch Reaktion einer Verbindung der Formel

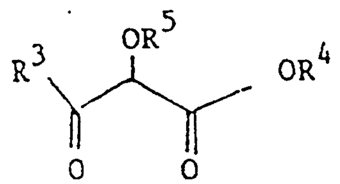

V,

worin $R^3$ und $R^5$ die in Anspruch 3 angegebene Bedeutung haben und $R^4$ $C_1$-$C_4$-Alkyl ist, mit einem Salz einer Verbindung der Formel

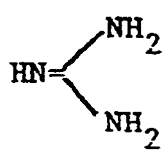

VI

erhalten wird.

8. Verfahren nach Anspruch 7, wobei das Salz eine Verbindung der Formel

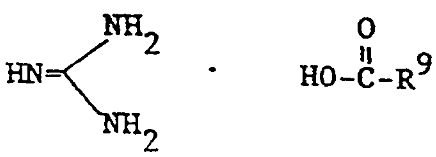

ist, worin $R^9$ ($C_1$-$C_4$)-Alkyl ist.

9. Verfahren zur Herstellung einer Verbindung der Formel

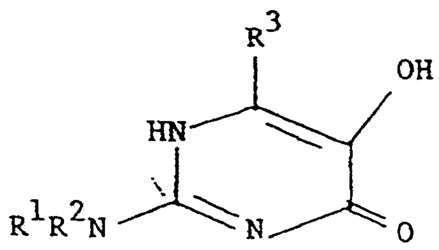

I

oder eines pharmazeutisch annehmbaren Salzes davon, worin $R^1$, $R^2$ und $R^3$ die in Anspruch 3 angegebene Bedeutung haben, wobei aus einer Verbindung der Formel

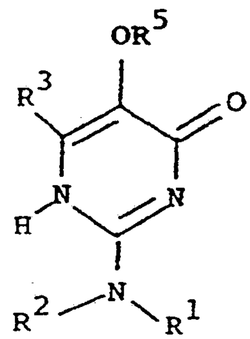

IX,

worin $R^1$, $R^2$ und $R^3$ obige Bedeutung haben und $R^5$ eine Schutzgruppe ist, die Schutzgruppe entfernt, und falls gewünscht, das Säureadditionssalz hergestellt wird.

10. Verfahren nach Anspruch 9, wobei aus der Verbindung der Formel IX die Schutzgruppe durch (a) Hydrolyse ; (b) Behandlung mit wässeriger Säure ; oder (c) Hydrierung entfernt wird.

11. Verfahren nach einem der vorhergehenden Ansprüche, wobei $R^1$ Wasserstoff ist, und $R^2$ lineares oder verzweigtes ($C_1$-$C_{15}$)-Alkyl oder ($C_7$-$C_{20}$)-Phenylalkyl, worin der Alkylteil linear oder verzweigt ist, ist.

12. Verfahren nach einem der Ansprüche 1 bis 10, wobei $R^2$ Cyclopentyl oder Cyclohexyl ist.

13. Verfahren nach einem der vorhergehenden Ansprüche, wobei die Verbindung der Formel I aus der Gruppe
2-Heptylamino-5-hydroxy-6-methyl-4-pyrimidon ;
2-(6-Methylheptyl-2-amino)-5-hydroxy-4-pyrimidon ;
2-[1-Methyl-3-phenyl)-propyl]amino-5-hydroxy-4-pyrimidon ; und
2-Benzylamino-5-hydroxy-4-pyrimidon ausgewählt ist.

## Revendications

### Revendications pour les Etats Contractants suivants : AT, BE, CH, DE, FR, GB, IT, LI, LU, NL, SE

1. Composé de formule

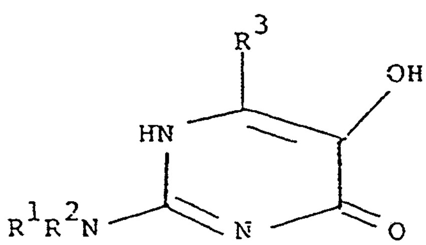

ou un de ses sels pharmaceutiquement acceptables,

formule dans laquelle $R^1$ représente l'hydrogène ; $R^2$ représente un groupe alkyle en $C_1$ à $C_{15}$, alcényle

en $C_3$ à $C_{15}$, phényle, phényle substitué, phénylalkyle en $C_7$ à $C_{20}$ ou phénylalkyle en $C_7$ à $C_{20}$ substitué, les substituants présents sur le groupe phényle substitué et le groupe phénylalkyle substitué consistant indépendamment en un ou deux groupes choisis entre des groupes fluoro, chloro, alkyle en $C_1$ à $C_3$, alkoxy en $C_1$ à $C_3$ et trifluorométhyle ; ou bien $R^1$ et $R^2$, conjointement avec l'atome d'azote auquel ils sont fixés, forment un groupe pyrrolidinyle, pyrrolidinyle substitué, pipéridyle ou pipéridyle substitué, les substituants présents sur les groupes pyrrolidinyle et pipéridyle substitués représentant indépendamment un groupe choisi entre des groupes alkyle en $C_1$ à $C_6$, phényle et phénylalkyle en $C_7$ à $C_9$ ; et $R^3$ représente l'hydrogène, un groupe alkyle en $C_1$ à $C_6$, phényle, phényle substitué, phénylalkyle en $C_7$ à $C_9$ ou phénylalkyle en $C_7$ à $C_9$ substitué, les substituants présents sur les groupes phényle substitués et phénylalkyle substitués représentant indépendamment un ou deux groupes choisis entre des groupes fluoro, chloro, alkyle en $C_1$ à $C_3$, alkoxy en $C_1$ à $C_3$ et trifluorométhyle, lesdits groupes alkyle, des portions de ces groupes alkyle et les portions alkyle d'autres groupes pouvant être linéaires, ramifiés, ou cycliques, sous réserve que, lorsque $R^1$ et $R^2$, conjointement avec l'atome d'azote auquel ils sont fixés, forment un groupe pipéridyle, $R^3$ ne représente pas un groupe méthyle.

2. Composé suivant la revendication 1, dans lequel $R^1$ représente l'hydrogène, $R^2$ représente un groupe alkyle en $C_1$ à $C_{15}$, linéaire ou ramifié, ou phénylalkyle en $C_7$ à $C_{20}$ dans lequel le groupement alkyle est linéaire ou ramifié ; et $R^3$ représente l'hydrogène ou un groupe méthyle.

3. Composé suivant la revendication 1, dans lequel $R^2$ représente un groupe cyclopentyle ou cyclohexyle.

4. Composé suivant la revendication 1, qui est
la 2-heptylamino-5-hydroxy-6-méthyl-4-pyrimidone ;
la 2-(6-méthylheptyl-2-amino)-5-hydroxy-4-pyrimidone ;
la 2-[(1-méthyl-3-phényl)-propyl]amino-5-hydroxy-4-pyrimidone ; ou
la 2-benzylamino-5-hydroxy-4-pyrimidone.

5. Composition pharmaceutique, qui comprend un composé suivant l'une quelconque des revendications précédentes ou un de ses sels pharmaceutiquement acceptables et un diluant ou support pharmaceutiquement acceptable.

6. Composé suivant l'une quelconque des revendications 1 à 4, ou un de ses sels pharmaceutiquement acceptables, destiné à être utilisé comme médicament.

7. Utilisation d'un composé suivant l'une quelconque des revendications 1 à 4, sans les réserves mentionnées, ou un de ses sels pharmaceutiquement acceptables, pour la production d'un médicament destiné au traitement de maladies pulmonaires, asthmatiques, allergiques, cardio-vasculaires, gastro-intestinales ou inflammatoires à médiation par les leucotriènes.

8. Utilisation suivant la revendication 7, dans laquelle les maladies sont l'arthrite, le psoriasis, la bronchite, l'hypertension pulmonaire, l'hypoxie pulmonaire, les ulcères peptiques, une affection intestinale inflammatoire non spécifique ou le spasme cardio-vasculaire.

**Revendications pour les Etats Contractants suivants : ES, GR**

1. Procédé de préparation d'un composé de formule

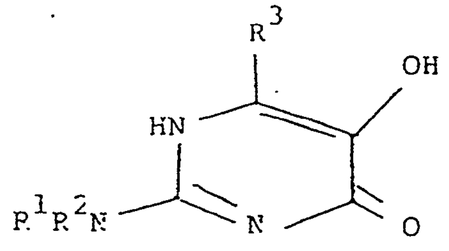

I

ou d'un de ses sels pharmaceutiquement acceptables,

formule dans laquelle $R^1$ représente l'hydrogène ; $R^2$ représente un groupe alkyle en $C_1$ à $C_{15}$, alcényle en $C_3$ à $C_{15}$, phényle, phényle substitué, phénylalkyle en $C_7$ à $C_{20}$ ou phénylalkyle en $C_7$ à $C_{20}$ substitué, les substituants présents sur le groupe phényle substitué et le groupe phénylalkyle substitué consistant indépendamment en un ou deux groupes choisis entre des groupes fluoro, chloro, alkyle en $C_1$ à $C_3$, alkoxy en $C_1$ à $C_3$ et trifluorométhyle ; ou bien $R^1$ et $R^2$, conjointement avec l'atome d'azote auquel ils sont fixés, forment un groupe pyrrolidinyle, pyrrolidinyle substitué, pipéridyle ou pipéridyle substitué, les substituants présents sur les groupes pyrrolidinyle et pipéridyle substitués représentant indépendamment un groupe choisi entre des groupes alkyle en $C_1$ à $C_6$, phényle et phénylalkyle en $C_7$ à $C_9$ ; et $R^3$ représente l'hydrogène, lesdits groupes alkyle, des portions de ces groupes alkyle et les portions alkyle d'autres groupes pouvant être linéaires, ramifiés ou

cycliques, consistant à faire réagir un composé de formule

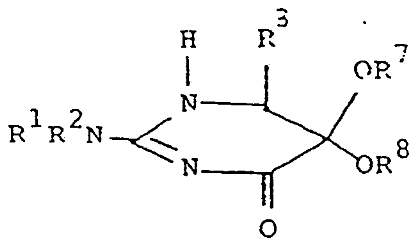

IV

dans laquelle $R^3$ représente l'hydrogène, $R^1$ et $R^2$ répondent aux définitions précitées, et $R^7$ et $R^8$ sont choisis indépendamment entre des groupes alkyle en $C_1$ à $C_4$, avec un catalyseur acide et, si cela est désiré, à préparer le sel d'addition d'acide.

2. Procédé suivant la revendication 1, dans lequel le composé de formule IV est obtenu par réaction d'un composé de formule

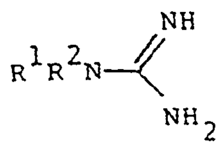

dans laquelle $R^1$ et $R^2$ répondent aux définitions précitées, avec un composé de formule

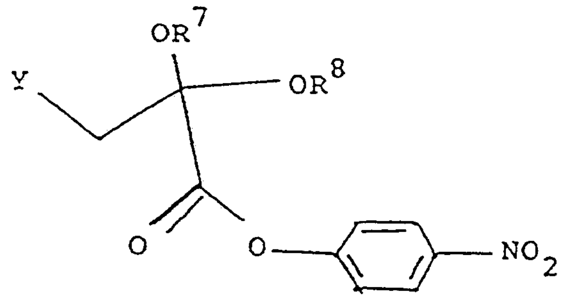

. III

dans laquelle $R^7$ et $R^8$ répondent aux définitions précitées et Y représente un groupe chloro, bromo ou iodo.

3. Procédé de préparation d'un composé de formule

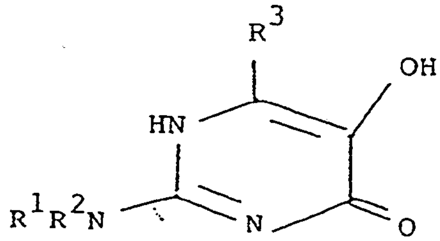

I

ou d'un de ses sels pharmaceutiquement acceptables,

formule dans laquelle $R^1$ et $R^2$ répondent aux définitions suivant la revendication 1 ; et $R^3$ représente l'hydrogène, un groupe alkyle en $C_1$ à $C_6$, phényle, phényle substitué, phénylalkyle en $C_7$ à $C_9$ ou phénylalkyle en $C_7$ à $C_9$ substitué, les substituants sur les groupes phényle substitué et phénylalkyle substitué représentant indépendamment un ou deux groupes choisis entre des groupes fluoro, chloro, alkyle en $C_1$ à $C_3$, alkoxy en $C_1$ à $C_3$ et trifluorométhyle, consistant à réduire, au moyen d'un agent réducteur du type hydrure dans un solvant anhydre, un composé de formule

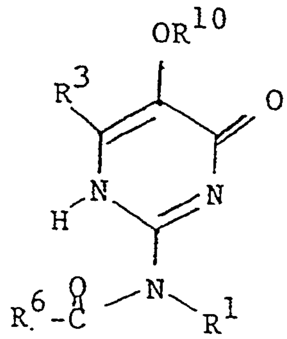

XIII

dans laquelle $R^1$ représente l'hydrogène, $R^3$ répond à la définition précitée, $R^{10}$ représente l'hydrogène ou $R^5$, $R^5$ représente un groupe protecteur et $R^6$ représente l'hydrogène, un groupe alkyle en $C_1$ à $C_{14}$, alcényle en $C_2$ à $C_{14}$, phénylalkyle en $C_6$ à $C_{19}$ ou phénylalkyle en $C_6$ à $C_{19}$ substitué, les substituants sur le groupe phénylalkyle substitué représentant indépendamment un ou deux groupes choisis entre des groupes fluoro, chloro, alkyle en $C_1$ à $C_3$, alkoxy en $C_1$ à $C_3$ et trifluorométhyle, et, si $OR^5$ représente un groupe qui n'est pas transformé par réduction en un groupe hydroxyle, à éliminer la protection du groupe $R^5$ après la réaction de réduction de sorte que le groupe $OR^5$ soit transformé en un groupe hydroxyle, et, si cela est désiré, à préparer le sel d'addition d'acide ; lesdits groupes alkyle, des portions de ces groupes alkyle et les portions alkyle d'autres groupes pouvant être linéaires, ramifiés ou cycliques.

4. Procédé suivant la revendication 3, dans lequel le composé de formule XIII est préparé par réaction d'un composé de formule

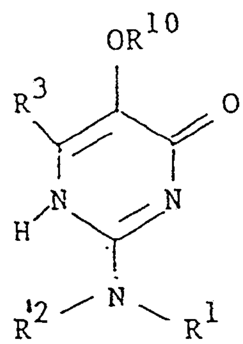

XIV

dans laquelle $R^1$, $R^3$ et $R^{10}$ répondent aux définitions suivant la revendication 3 et $R^{12}$ représente H, avec un composé de formule

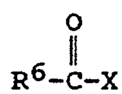

X

dans laquelle $R^6$ répond à la définition suivant la revendication 3 et X représente un groupe chloro ou bromo.

5. Procédé suivant la revendication 4, dans lequel le composé de formule XIV, dans laquelle $R^{10}$ représente un groupe $R^5$, est préparé par réaction d'un composé de formule

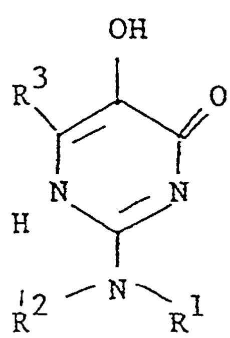

VIII

dans laquelle $R^1$ et $R^3$ répondent aux définitions suivant la revendication 3 et $R^{12}$ représente H, avec un réactif capable de remplacer l'hydrogène dans le groupe hydroxyle par un groupe protecteur.

6. Procédé suivant la revendication 5, dans lequel le composé de formule XIV, dans laquelle $R^{10}$ représente un groupe $R^5$, est préparé par réaction du composé de formule VIII avec l'anhydride acétique et une base.

7. Procédé suivant l'une quelconque des revendications 4 à 6, dans lequel le composé de formule XIV est obtenu par réaction d'un composé de formule

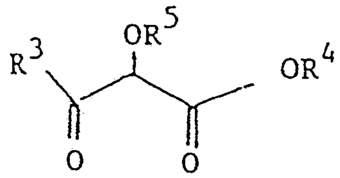

V

dans laquelle $R^3$ et $R^5$ répondent aux définitions suivant la revendication 3 et $R^4$ représente un groupe alkyle en $C_1$ à $C_4$, avec un sel d'un composé de formule

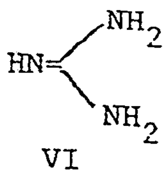

VI

8. Procédé suivant la revendication 7, dans lequel le sel est un composé de formule

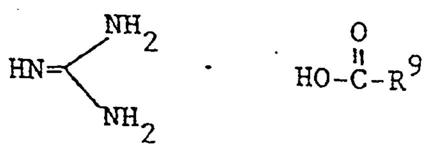

dans laquelle $R^9$ représente un groupe alkyle en $C_1$ à $C_4$.

9. Procédé de préparation d'un composé de formule

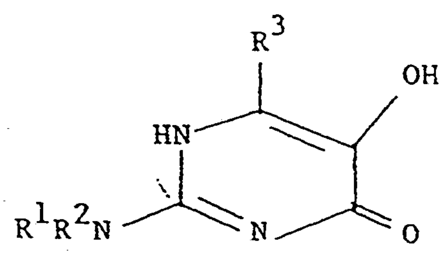

I

ou d'un de ses sels pharmaceutiquement acceptables,

formule dans laquelle $R^1$, $R^2$ et $R^3$ répondent aux définitions suivant la revendication 3, consistant à éliminer la protection d'un composé de formule

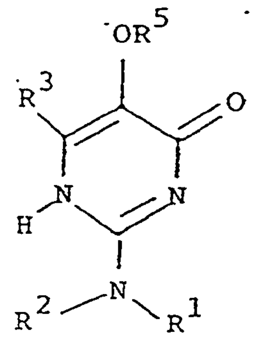

IX

dans laquelle $R^1$, $R^2$ et $R^3$ répondent aux définitions précitées et $R^5$ représente un groupe protecteur, et, si cela est désiré, à préparer le sel d'addition d'acide.

10. Procédé suivant la revendication 9, dans lequel le composé de formule IX est débarrassé de sa protection par (a) hydrolyse ; (b) traitement avec une solution aqueuse d'un acide ; ou (c) hydrogénation.

11. Procédé suivant l'une quelconque des revendications précédentes, dans lequel $R^1$ représente l'hydrogène et $R^2$ représente un groupe alkyle en $C_1$ à $C_{15}$, linéaire ou ramifié, ou phénylalkyle en $C_7$ à $C_{20}$ dans lequel le groupement alkyle est linéaire ou ramifié.

12. Procédé suivant l'une quelconque des revendications 1 à 10, dans lequel $R^2$ représente un groupe cyclopentyle ou cyclohexyle.

13. Procédé suivant l'une quelconque des revendications précédentes, dans lequel le composé de formule I est choisi dans le groupe comprenant

la 2-heptylamino-5-hydroxy-6-méthyl-4-pyrimidone ;

la 2-(6-méthylheptyl-2-amino)-5-hydroxy-4-pyrimidone ;

la 2-[1-méthyl-3-phényl)-propyl]amino-5-hydroxy-4-pyrimidone ; et

la 2-benzylamino-5-hydroxy-4-pyrimidone.